# FASCICULE DE BREVET EUROPEEN

(11) **EP 4 288 029 B1**
(45) Date de publication et mention de la délivrance du brevet: **25.12.2024**
(21) Numéro de dépôt: 22701660.7
(22) Date de dépôt: 01.02.2022
(51) Int. Cl.: A61K 9/00, A61K 9/06, A61K 31/23, A61K 31/231, A61K 31/232, A61K 47/36, A61P 25/22, A61P 25/24, A61P 43/00, A61P 25/00

(54) **GEL DIFFUSEUR DE PHEROMONES**
PHEROMON-DIFFUSIONSGEL
PHEROMONE DIFFUSING GEL

(30) Priorité: 02.02.2021 FR 2100989
(43) Date de publication de la demande: 13.12.2023
(73) Titulaire: VIRBAC, 06516 Carros (FR)
(72) Inventeur: GIRARDIN, Aurélie, 06700 Saint Laurent Du Var (FR); MONGINOUX, Patricia, 06270 Villeneuve Loubet (FR); DA SILVA PINTO, Vitor Manuel, 2900-692 Setúbal (PT); RAMOS DA SILVA RAÍNHO, Vera Mónica, 2625-594 Vialonga (PT); MENDES RODRIGUES, Cátia Filipa, 2100-528 Fajarda (PT); NOVO DE CARVALHO, Cindy, 4435-599 Gondomar (PT); MACHADO PARRULA SILVA, Maria Helena, 2870-052 Montijo (PT)
(74) Mandataire: Gevers & Orès
(86) Numéro de dépôt international: PCT/EP2022/052271
(87) Numéro de publication internationale: WO 2022/167389

(56) Documents cités:
- EP-B1- 2 954 886
- WO-A1-01/30146
- WO-A1-2009/144321
- WO-A1-2015/140631
- WO-A1-2016/103133
- WO-A1-98/35567
- GB-A- 2 345 635

## Description

La présente invention a pour objet des dispositifs diffuseurs pour chiens et pour chats contenant des formulations capables de diffuser passivement des compositions sémiochimiques.

La présente invention a en particulier pour objet des dispositifs contenant des formulations capables de diffuser des compositions sémiochimiques efficaces sur les chiens ou les chats pendant une durée supérieure aux dispositifs connus de l'art antérieur et ne nécessitant pas d'être reliés à une alimentation électrique pour fonctionner. La présente invention a aussi pour objet les formulations de diffusion contenues dans de tels dispositifs, en particulier les gels diffuseurs comprenant les compositions sémiochimiques efficaces sur les chiens et les chats.

On connait dans l'art antérieur des dispositifs capables de diffuser passivement des compositions sémiochimiques. Les compositions sémiochimiques sont des composés ou des mélanges de composés naturellement sécrétés par les animaux pour communiquer au sein d'une même espèce. Par exemple, la chienne sécrète pendant la lactation une phéromone pour rassurer ses chiots lorsqu'ils sont près d'elle. De la même façon, le chat sécrète, au niveau de sa face, des phéromones faciales qu'il dépose dans les endroits connus pour marquer un territoire familier.

De nombreuses études ont été réalisées pour identifier ces mélanges de composés et les reproduire synthétiquement.

De tels mélanges de composés sont par exemple indiqués pour aider les chiens à mieux gérer les situations génératrices de stress (situations de séparation, sevrage, transport, peur des bruits, des feux d'artifice etc.) et leur permettre de mieux s'adapter aux situations nouvelles.

Les produits pour animaux de compagnie, en particulier les chiens et les chats, concernés sont par exemple des sprays, des diffuseurs actifs (prises électriques chauffant une composition sémiochimique dispersée ou solubilisée dans un solvant organique) et des colliers. Par exemple, EP2954868B1 décrit une composition Adapil^{®} sous forme de spray comprenant une mousse de polyuréthane imprégnée de la composition phéromonale, cette composition étant présente dans un dispositif conçu pour être activé à proximité du nez de l'animal, par exemple au moment du transport, afin d'obtenir un effet calmant immédiat, sans besoin de chaleur. WO2016103133A1 divulgue également le produit commercial Feliway^{®}, qui contient un analogue synthétique de la fraction F3 des phéromones faciales de chat, solubilisé dans de l'éthanol.

Les diffuseurs actifs de l'art antérieur (ADAPTIL^{®} Calm pour chien) comprennent un réservoir contenant une composition sémiochimique dispersée ou solubilisée dans un solvant organique dans laquelle trempe une mèche. Lorsque le dispositif est branché sur le courant, la mèche est chauffée ce qui entraîne la diffusion de la composition sémiochimique dans l'environnement de l'animal à travers cette mèche ; la composition sémiochimique est constituée d'un mélange d'esters méthyliques d'acides gras ou d'un mélange d'esters méthyliques d'acides gras et d'acides gras. Ce diffuseur actif est efficace pendant 4 semaines.

Un inconvénient majeur de ce type de dispositif utilisé pour le chien ou le chat est qu'il nécessite de disposer d'une alimentation électrique pour l'activer et mettre en oeuvre la diffusion de la composition sémiochimique. Aussi il ne peut pas être utilisé dans certains lieux dans lesquels évoluent les chiens et les chats.

Des dispositifs de diffusion passive de composition sémiochimique ont été utilisés pour des animaux d'élevage. Chez les porcs, par exemple, il existe des blocs comprenant des PAP ou « Pig Appeasing Pheromon » ou encore phéromone apaisante de porc, que l'on utilise dans les fermes d'élevage en les positionnant à 1,5 mètre de hauteur dans une enceinte d'élevage pendant toute leur durée d'utilisation afin de réduire l'agressivité et préserver le bien-être des groupes de porcs sevrés (Temple et al., Porcine Health Management 2, 13 (2016)) ; cependant, la composition des blocs à relargage lent contenus dans ces dispositifs pour animaux d'élevage n'est pas décrite et leur efficacité est limitée à 6 semaines.

Les Inventeurs ont mis au point des formulations de diffusion contenues dans des dispositifs portables et déplaçables à volonté sans restrictions, sous forme de gel diffuseur comprenant une composition sémiochimique comprenant un mélange de dérivés volatils d'acide gras ; ce gel diffuseur permet une diffusion passive efficace de la composition sémiochimique dans l'environnement ; cette formulation permet ainsi de s'affranchir de la nécessité d'une alimentation électrique pour l'utilisation d'un dispositif diffuseur intégrant la formulation, et elle peut ainsi être transportée et utilisée en tout lieu. En outre, elle présente une durée d'efficacité prolongée par rapport aux diffuseurs de l'art antérieur.

Par ailleurs, la composition sémiochimique formulée dans cette formulation de diffusion montre une bonne stabilité malgré la fragilité à l'humidité et à la lumière de ses composants et le dispositif de diffusion passive est constitué d'une enceinte qui permet avantageusement de contrôler la diffusion de la composition sémiochimique.

La présente invention a donc pour objet l'amélioration des dispositifs de l'art antérieur, en particulier des diffuseurs pour chiens et/ou pour chats, contenant une composition sémiochimique comprenant un mélange de dérivés volatils d'acide gras.

Sauf indication contraire, tous les pourcentages sont indiqués en masse d'un composant par rapport à la masse totale des composants indiqués (m/m).

Un premier objet de la présente invention est un gel diffuseur passif comprenant :
- une matrice gélifiée polysaccharidique,
- une composition sémiochimique apaisante pour chiens et/ou pour chats comprenant un mélange de dérivés volatils d'acide gras, ledit dérivé volatil d'acide gras étant un ester éthylique ou méthylique d'acide gras,
- un agent solubilisant de ladite composition sémiochimique dans ladite matrice gélifiée,
- un acide organique ayant un pKa supérieur à 3,
- optionnellement, un conservateur,
- optionnellement, un colorant,
- optionnellement, un agent amérisant.

La **matrice gélifiée polysaccharidique** est un gel aqueux de nature polysaccharidique, elle est ainsi composée d'un gélifiant polysaccharidique et d'eau ; le gélifiant polysaccharidique peut être du κ-carraghénane, 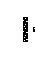 -carraghénane, λ-carraghénane, agarose (agar), gomme gellane, gomme d'amylose, gomme de curdlane, alginate, gomme de xanthane, chitosan et gomme de rhamsane désacétylé ou un mélange de ces gélifiants polysaccharidiques.

Selon un mode de réalisation particulier, la matrice gélifiée est préparée à partir de carraghénane ; de préférence, selon ce mode de réalisation, le carraghénane est présent en une teneur comprise entre 0,5 à 5%, préférentiellement entre 1 et 2%, en poids par rapport au poids total du gel diffuseur.

Afin d'obtenir un gel ayant une viscosité appropriée qui présente les avantages de l'invention en terme de stabilisation de la composition sémiochimique et de relargage contrôlé et continu de quantités adéquates des composés sémiochimiques, il est nécessaire d'ajuster le pH du gel, en particulier à l'aide d'un **acide organique** ayant un pKa supérieur à 3. L'acide organique peut être tout acide organique mais on utilisera de préférence l'acide citrique qui s'avère être particulièrement adapté aux compositions sémiochimiques selon l'invention. L'acide organique ayant un pKa supérieur à 3, en particulier l'acide citrique, est généralement présent à une teneur comprise entre 0,01 et 0,5%, de préférence de l'ordre de 0,05%, en masse de la masse totale du gel diffuseur.

La **composition sémiochimique** employée est une composition sémiochimique apaisante pour chiens et/ou pour chats comprenant un mélange de dérivés volatils d'acides gras.

Au sens de la présente invention, une composition sémiochimique apaisante qualifie un mélange de substances chimiques ayant valeur de signal entre des êtres vivants d'origine naturelle, par exemple isolée d'un être vivant, ou d'origine synthétique, par exemple obtenue par mélange de dérivés volatils d'un acide gras susceptible de produire un effet relaxant ou apaisant sur l'animal.

Au sens de la présente invention, on entend par « acide gras » un acide monocarboxylique ou dicarboxylique à chaîne hydrocarbonée, saturé ou insaturé, linéaire ou ramifié ayant entre 4 et 22 atomes de carbones. Avantageusement, l'acide gras est choisi dans le groupe constitué de l'acide oléique, l'acide palmitique, l'acide azélaïque, l'acide pimélique, l'acide caprique, l'acide laurique, l'acide myristique, l'acide palmitoléique, l'acide linoléique, l'acide stéarique, l'acide arachidonique, l'acide n-butyrique, l'acide isobutyrique, l'acide α-méthylbutyrique, l'acide caproïque, l'acide pivalique, l'acide γ-linoléique, l'acide eicosapentanoïque, l'acide pentadécanoïque, l'acide tridécanoïque, ou l'acide docosahexanoïque.

Les dérivés d'acides gras volatils sont l'ester méthylique ou éthylique d'acide gras. De préférence, l'ester d'acide gras est l'ester méthylique d'acide gras.

De manière avantageuse, la composition sémiochimique apaisante pour chiens et/ou pour chats comprend donc un mélange de dérivés volatiles d'acides gras où l'acide gras est choisi dans le groupe constitué de l'acide oléique, l'acide palmitique, l'acide azélaïque, l'acide pimélique, l'acide caprique, l'acide laurique, l'acide myristique, l'acide palmitoléique, l'acide linoléique, l'acide stéarique, l'acide arachidonique, l'acide n-butyrique, l'acide isobutyrique, l'acide α-méthylbutyrique, l'acide caproïque, l'acide pivalique, l'acide γ-linoléique, l'acide eicosapentanoïque, l'acide pentadécanoïque, l'acide tridécanoïque, ou l'acide docosahexanoïque, avantageusement choisi dans le groupe constitué de l'acide oléique, l'acide palmitique, l'acide laurique, l'acide myristique, l'acide linoléique, l'acide stéarique, et l'acide pentadécanoïque.

De manière plus avantageuse, la composition sémiochimique apaisante pour chiens et/ou pour chats comprend ou consiste en un mélange de dérivés volatiles d'acides gras choisis dans le groupe constitué de l'oléate de méthyle, du palmitate de méthyle, de l'azélate de méthyle, du pimélate de méthyle, du caprate de méthyle, du laurate de méthyle, du myristate de méthyle, du palmitoléate de méthyle, du linoléate de méthyle, du stéarate de méthyle, de l'arachidonate de méthyle, du n-butyrate de méthyle, de l'isobutyrate de méthyle, de l'α-méthylbutyrate de méthyle, du caproate de méthyle, du pivalate de méthyle, du γ-linoléate de méthyle, de l'eicosapentanoate de méthyle, du pentadécanoate de méthyle, du tridécanoate de méthyle, du docosahexanoate de méthyle, avantageusement choisi dans le groupe constitué de l'oléate de méthyle, du palmitate de méthyle, du laurate de méthyle, du myristate de méthyle, du linoléate de méthyle, du stéarate de méthyle et du pentadécanoate de méthyle.

Les dérivés volatils d'acides gras et leurs proportions relatives dans la composition sémiochimique apaisante dépendent de l'animal cible.

Lorsque l'animal cible est un chien, la composition sémiochimique apaisante pour chiens et/ou pour chats peut être choisie parmi celles décrites dans la demande internationale WO2009144321.

Des mélanges particuliers d'esters, de préférence d'esters méthyliques, des acides gras destinés à apaiser les chiens sont les esters des acides gras suivants :
- un mélange **A** d'acide oléique et d'acide n-butyrique ;
- un mélange **B** d'acide oléique, d'acide palmitique et d'acide linoléique ;
- un mélange **C** d'acide oléique, d'acide palmitique, d'acide linoléique et d'acide palmitoléique ;
- un mélange **D** d'acide caprique, d'acide laurique, d'acide myristique, d'acide palmitoléique, d'acide palmitique, d'acide linoléique et d'acide oléique ;
- un mélange **E** d'acide oléique, d'acide palmitique, d'acide linoléique et d'acide myristique ;
- un mélange **F** d'acide oléique, d'acide palmitique, d'acide linoléique, d'acide laurique et d'acide myristique ;
- un mélange **G** d'acide oléique, d'acide palmitique, d'acide linoléique, d'acide myristique et d'acide pentadécanoïque ;
- un mélange **H** d'acide oléique, d'acide palmitique, d'acide linoléique, d'acide myristique, d'acide pentadécanoïque et d'acide stéarique ;
- un mélange **I** d'acide oléique, d'acide palmitique, d'acide linoléique, d'acide myristique, d'acide laurique et d'acide pentadécanoïque ;
- un mélange **J** d'acide laurique, d'acide myristique, d'acide pentadécanoïque, d'acide palmitique, d'acide stéarique, d'acide oléique, et d'acide linoléique ;
- un mélange **K** d'acide oléique, d'acide azélaïque, d'acide pimélique et d'acide palmitique.

Des mélanges d'esters, de préférence les esters méthyliques, d'acides gras destinés à apaiser les chiens sont des esters des acides gras suivants :
- mélange **L** comprenant ou consistant en environ 55-65% d'acide oléique, et 45-35% d'acide palmitique ;
- mélange **M** comprenant ou consistant en environ 45% d'acide oléique, 16% d'acide azélaïque, 18% d'acide pimélique, et 21% d'acide palmitique ;
- mélange **N** comprenant ou consistant en environ 30% d'acide palmitique, 30% d'acide oléique, et 40% d'acide linoléique ;
- mélange **O** comprenant ou consistant en environ 30% d'acide palmitique, 40% d'acide linoléique, 10% d'acide palmitoléique, et 20% d'acide oléique.

Un mélange particulier **P** d'esters d'acides gras destiné à apaiser les chiens comprend ou consiste en environ 35% d'oléate de méthyle, environ 2% de laurate de méthyle, environ 13% de stéarate de méthyle, environ 21% de linoléate de méthyle, environ 5% de myristate de méthyle, environ 4% de pentadécanoate de méthyle, et environ 20% de palmitate de méthyle.

Les Inventeurs de la présente invention ont par ailleurs mis en évidence une composition sémiochimique comprenant ou consistant en un mélange de dérivés volatils d'acide gras ne comprenant pas de pentadécanoate de méthyle conduisait à un effet similaire sur le chien à celui du mélange **P.** L'acide pentadécanoïque étant un acide gras relativement rare naturellement, dont l'origine principale est la fraction grasse du lait de vache où il est présent en quantité faible (1,2%), il est difficile d'en obtenir à un coût raisonnable. La découverte que l'absence de cet acide gras ne diminue pas l'efficacité apaisante d'une composition sémiochimique permet donc de réduire les coûts associés à la fabrication des dispositifs selon l'invention. Un autre mélange particulier **Q** d'esters d'acides gras destiné à apaiser les chiens selon l'invention comprend ou consiste en entre 32% et 38% d'oléate de méthyle, entre 1 et 3% de laurate de méthyle, entre 13 et 16% de stéarate de méthyle, entre 18 et 24% de linoléate de méthyle, entre 3 et 7% de myristate de méthyle et entre 18 et 24% de palmitate de méthyle.

Un mélange plus particulier **R** d'esters d'acides gras destinés à apaiser les chiens selon l'invention comprend ou consiste en environ 35% d'oléate de méthyle, environ 2% de laurate de méthyle, environ 15,5% de stéarate de méthyle, environ 21% de linoléate de méthyle, environ 5% de myristate de méthyle et environ 21% de palmitate de méthyle.

Lorsque l'animal cible est un chat, la composition sémiochimique apaisante peut être choisie parmi celles décrites dans la demande internationale WO2015140631.

La composition sémiochimique apaisante est par exemple une phéromone apaisante pour chat qui est dérivée de sécrétions cutanées provenant de la zone située entre les omoplates et les oreilles des chats.

La composition sémiochimique destinée à apaiser les chats comprend ou consiste en avantageusement du palmitate de méthyle, du linoléate de méthyle, de l'oléate de méthyle, du stéarate de méthyle, du laurate de méthyle et du myristate de méthyle.

Un mélange particulier **S** d'esters d'acides gras destiné à apaiser les chats comprend ou consiste en entre 15% et 25% de palmitate de méthyle, entre 10% et 27% de linoléate de méthyle, entre 25% et 35% d'oléate de méthyle, entre 5% à 15% de stéarate de méthyle, entre 5% à 15% de laurate de méthyle et entre 5% à 15% de myristate de méthyle.

Un autre mélange particulier **T** d'esters d'acides gras destiné à apaiser les chats comprend ou consiste en entre 19% et 26% de palmitate de méthyle, entre 14% et 20% de linoléate de méthyle, entre 26% et 32% d'oléate de méthyle, entre 8% et 14% de stéarate de méthyle, entre 8% et 14% de laurate de méthyle et entre 8% et 14% de myristate de méthyle.

Un mélange encore plus particulier **U** comprend ou consiste en 22% de palmitate de méthyle, 17% de linoléate de méthyle, 28% d'oléate de méthyle, 11% méthyle stéarate, 11% de laurate de méthyle et 11% de myristate de méthyle.

Une autre composition sémiochimique **V** pouvant être utilisée dans les dispositifs selon l'invention est décrite dans le brevet US10227321, dans laquelle au moins un dérivé volatil d'acide gras est un ester de formule suivante : 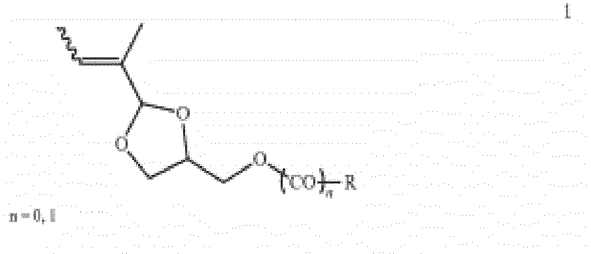 dans laquelle n=0 et R est un alkyl linéaire saturé ou insaturé comprenant de 9 à 30 atomes de carbone ou n=1 et R est un alkyl linéaire saturé ou insaturé comprenant de 8 à 22 atomes de carbone.

La quantité de composition sémiochimique dans le dispositif peut varier dans de larges proportions. La proportion de composition sémiochimique est typiquement comprise entre 0,1% et 25% en masse de la masse totale du gel diffuseur. Avantageusement, la composition sémiochimique représente de 1 à 10% en masse, plus avantageusement de 3 à 8% en masse, de préférence environ 6% de la masse totale du gel diffuseur.

Par « environ x% », on entend au sens de la présente invention une variation de plus ou moins 0,2% par rapport au pourcentage indiqué.

La composition sémiochimique étant constituée de molécules non miscibles à l'eau et en raison de la forte teneur en eau du gel, le gel diffuseur selon l'invention comprend au moins un **agent solubilisant** de la composition sémiochimique dans la matrice gélifiée, afin d'éviter la formation de deux phases, la première étant constituée d'eau et la deuxième de la composition sémiochimique (démixtion).

L'agent solubilisant a également pour rôle de faciliter l'évaporation des composants de la composition sémiochimique. Cette évaporation influence le relargage de la composition sémiochimique et donc l'efficacité du dispositif selon l'invention.

L'agent solubilisant peut être au moins un tensioactif, au moins un solvant organique miscible à l'eau ou un mélange d'au moins un tensioactif et d'au moins un solvant organique miscible à l'eau.

Le tensioactif peut être choisi parmi les tensioactifs anioniques, non-ioniques, cationiques ou amphotères.

Les tensioactifs anioniques comprennent les acylaminoacides (et sels); les acides carboxyliques (et sels), les acides phosphoriques (et sels); les acides sulfoniques (et sels), les alkylaryl sulfonates, les alkyl sulfonates, les alkyl sulfosuccinates (et sels) et les esters d'acide sulfurique, tels que les alkyl éther sulfates, les alkyl sulfates, les alkylphénols éthoxylés et les alkylphénols sulfatés.

Les tensioactifs non-ioniques comprennent les alcools aliphatiques éthoxylés; les polyoxyéthylènes; les esters carboxyliques; les esters de polyéthylène glycol; les esters d'anhydrosorbitol et leurs dérivés éthoxylés; les esters de glycol d'acides gras; les amides carboxyliques; les amides d'acide gras polyoxyéthylénés.

Les tensioactifs cationiques comprennent principalement des sels d'ammonium quaternaire alkyl à longue chaîne.

Les tensioactifs amphotères comprennent les tensioactifs zwitterioniques tels que les bétaïnes (par exemple les alkyl bétaïnes, les alkylamido bétaïnes, les sulfobétaïnes, les sulfitobétaïnes, les sulfatobétaïnes, les phosphinates bétaïnes, les phosphonates bétaïnes, les phosphito bétaïnes, les phosphate bétaïnes, les sulfonium bétaïnes et les phosphénium bétaïnes).

Le solvant organique miscible à l'eau peut être tout solvant miscible à l'eau dans lequel la composition sémiochimique est soluble.

Parmi les solvants organiques miscibles à l'eau, on peut citer les alcools tels que le méthanol, l'éthanol, le 1-propanol, le 2-propanol, le 1-butanol, le 2-butanol et le tert-butanol ; les cétones telles que l'acétone ; les éthers tels que le dioxane ou le diméthoxyéthane ; les sulfoxydes tels que le diméthyl sulfoxyde ; les amides tels que le diméthylformamide ou N-Methyl-2-pyrrolidone ; les nitriles tels que l'acétonitrile ; les polyols tels que le 1,2-Butanediol, le 1,3-Butanediol, le 1,3-Propanediol, le1,4-Butanediol, le 1,5-butanediol, l'éthylène glycol, le propylène glycol, le glycérol ou le triéthylène glycol.

Avantageusement, l'agent solubilisant est un solvant organique miscible à l'eau, en particulier choisi parmi les alcools en C1 à C4 et les polyols. De préférence, l'alcool en C1 à C4 est le 1-propanol ou le 2-propanol, plus préférentiellement le 2-propanol.

On utilise une quantité aussi faible que possible de l'agent solubilisant. Généralement, une teneur en agent solubilisant comprise entre 1 et 10% en masse de la masse totale du gel diffuseur permet d'obtenir un gel homogène et stable dans le temps, pour lequel aucune démixtion n'est observée.

Lorsque l'agent solubilisant est un alcool en C1 à C4 ou un polyol, en particulier le 1-propanol ou le 2-propanol, plus préférentiellement le 2-propanol, il est présent à une teneur comprise entre 1 et 10%, avantageusement entre 2 et 8 %, de préférence de l'ordre de 4%, en masse de la masse totale du gel diffuseur.

Tout **conservateur,** en particulier les conservateurs de type biocide, peut être ajouté au gel diffuseurs ; cette famille de produit est bien connue de l'homme du métier ; elle comprend notamment les isothiazolinones (benzoisothiazolinone, isothiazolinone, méthylisothiazolinone, méthylchloroisothiazolinone, n-octyl-isothiazolinone...), les ammoniums quaternaires (benzalkonium).

Selon un mode de réalisation particulier, le conservateur est le benzoisothiazolinone et est présent à une teneur comprise entre 0,05 et 1%, de préférence de l'ordre de 0,18%, en masse de la masse totale du gel diffuseur.

En complément, la stabilité du gel diffuseur selon la présente invention et en particulier la stabilité des composants de la composition sémiochimique, peut encore être améliorée en incorporant des **antioxydants.** Parmi les antioxydants, on peut notamment citer les composés phénoliques tels que l'hydroxyanisole de butyle (BHA), l'hydroxytoluène de butyle (BHT), l'acide gallique, le gallate de propyle et le pyrogallol, les hydroquinones telles que la tert-butylhydroquinone (TBHQ) et la di-tert-butylhydroquinone (DTBHQ), les tocophérols, les amines telles que l'octyl butyl diphénylamine (OBPA), la poly(1,2-dihydro-2,2,4-triméthylquinoléine (Orox PK), la N,N'-phényl-p-phénylènediamine (DPD) et l'éthoxyquine, et un mélange de ces antioxydants. De tels composés sont bien connus de l'Homme du Métier et peuvent être choisis sans effort excessif ; de préférence, l'antioxydant utilisé est la TBHQ. A titre indicatif, la quantité d'antioxydants utilisée peut être comprise entre 0,001 et 0,1%.

Afin de rendre l'aspect visuel du gel diffuseur plus attrayant, tout **colorant** peut lui être ajouté.

Enfin, pour éviter l'ingestion accidentelle du gel diffuseur, par un enfant ou un animal par exemple, il peut lui être ajouté un **agent amérisant** ; ceux-ci sont connus de l'homme du métier comme le benzoate de denatonium, ce composé peut être ajouté en une teneur comprise entre 0,05 à 0,15% en masse par rapport à la masse totale du gel diffuseur.

La présente invention se rapporte encore à un gel diffuseur comprenant :
- une matrice gélifiée polysaccharidique ;
- de 1 à 10% d'une composition sémiochimique apaisante pour chiens et ou pour chats comprenant un mélange de dérivés volatils d'acide gras telle que définie ci-dessus, de préférence environ 6% ; en particulier, la composition sémiochimique peut comprendre un mélange d'esters d'acides gras choisis parmi le laurate, de myristate, de palmitate, de stéarate, d'oléate et de linoléate, et éventuellement de pentadécanoate ;
- de 1 à 10% d'un agent solubilisant de ladite composition sémiochimique dans ladite matrice gélifiée, de préférence environ 4% ;
- entre 0,1 et 1% d'un acide organique ayant un pKa supérieur à 3, en particulier l'acide citrique ;
- optionnellement, de 0,1 à 0,5% d'un conservateur ;
- optionnellement, un colorant ;
- optionnellement, un agent amérisant.

Dans un mode de réalisation spécifique, le dispositif selon la présente invention comprend :
- une matrice gélifiée constituée d'un gel aqueux de carraghénane ;
- de 1 à 10% d'une composition sémiochimique telle que définie ci-dessus, de préférence environ 6% ; en particulier, composition sémiochimique peut comprendre un mélange d'esters d'acides gras choisis parmi le laurate, de myristate, de palmitate, de stéarate, d'oléate et de linoléate, et éventuellement de pentadécanoate ;
- de 1 à 10% d'un agent solubilisant de ladite composition sémiochimique dans ladite matrice gélifiée choisi parmi les alcools en C1 à C4, de préférence environ 4% ;
- entre 0,1 et 1% d'acide citrique ;
- optionnellement, de 0,1 à 0,5% d'un conservateur de la famille des isothiazolinones ;
- optionnellement, un colorant ;
- optionnellement, de benzoate de dénathorium à titre d'agent amérisant.

Dans un mode de réalisation spécifique, le dispositif selon la présente invention comprend :
- une matrice gélifiée comprenant un gel aqueux de carraghénane ;
- de 1 à 10% d'une composition sémiochimique, ladite composition sémiochimique étant un mélange d'esters d'acides gras comprenant entre 32% et 38% d'oléate de méthyle, entre 1 et 3% de laurate de méthyle, entre 13 et 16% de stéarate de méthyle, entre 18 et 24% de linoléate de méthyle, entre 3 et 7% de myristate de méthyle et entre 18 et 24% de palmitate de méthyle, de préférence environ 6% ;
- de 2 à 10% d'un agent solubilisant de ladite composition sémiochimique dans ladite matrice gélifiée choisi parmi les alcools en C1 à C4 ;
- entre 0,1 et 1% d'acide citrique ;
- de 0,1 à 0,5% d'un conservateur de la famille des isothiazolinones ;
- un colorant ;
- du benzoate de dénatonium à titre d'agent amérisant.

Un deuxième objet de la présente invention est un **dispositif diffuseur** contenant un gel diffuseur tel que décrit selon le premier objet de l'invention, permettant la diffusion dans l'environnement du chien et/ou du chat de la composition sémiochimique qui va alors produire son effet apaisant sur l'animal.

Le dispositif diffuseur est un récipient susceptible de contenir un gel diffuseur comprenant:
- une matrice gélifiée polysaccharidique,
- une composition sémiochimique apaisante pour chiens et/ou pour chats comprenant un mélange de dérivés volatils d'acide gras,
- un agent solubilisant de ladite composition sémiochimique dans ladite matrice gélifiée,
- un acide organique ayant un pKa supérieur à 3,
- optionnellement, un conservateur,
- optionnellement, un colorant,
- optionnellement, un agent amérisant.

Le dispositif diffuseur est un récipient destiné à contenir le gel diffuseur et à permettre le relargage contrôlé de la composition sémiochimique. Il est susceptible d'avoir n'importe quelle forme et comporte nécessairement au moins une zone d'échange du gel diffuseur avec l'air, afin que la composition sémiochimique apaisante pour chiens et/ou pour chats diffuse dans l'air autour du dispositif diffuseur. Ainsi, le positionnement du dispositif diffuseur à proximité du chien et/ou du chat permet au chat et/ou au chien d'être en contact avec la composition sémiochimique apaisante. Les dispositifs diffuseurs sont portables ou transportables ou encore nomades, c'est-à-dire qu'ils peuvent être transportés et positionnés par le propriétaire du chien ou du chat de places en places dans les lieux dans lesquels les animaux domestiques évoluent afin de produire leur effet. Les dispositifs diffuseurs selon l'invention seront principalement disposés dans des lieux clos dans lesquels le chien ou le chat évolue afin d'éviter une dispersion superflue des composés sémiochimiques par le vent. Il s'agit par exemple de pièces d'habitation, d'un véhicule (une voiture) ou encore d'une salle d'attente chez un vétérinaire.

La zone d'échange du gel diffuseur avec l'air est préférentiellement située sur le dessus du récipient afin d'éviter tout risque d'écoulement du gel diffuseur hors du récipient.

La zone d'échange du gel diffuseur avec l'air est avantageusement constituée d'une paroi du récipient dans laquelle sont ménagées une ou une pluralité d'ouvertures par lesquelles la composition sémiochimique peut diffuser. Les ouvertures peuvent avoir des formes géométriques simples telles que des ronds, des carrés, des losanges, des rectangles et des triangles ou avoir des formes ayant une fonction esthétique telle que la tête de l'animal auquel est destiné le dispositif, la silhouette du corps de l'animal auquel est destiné le dispositif ou encore la patte de l'animal auquel est destiné le dispositif. Ce type d'indication visuelle, lorsqu'il identifie l'animal cible, le chien ou le chat, peut constituer une aide précieuse pour un propriétaire âgé dans le bon usage du dispositif diffuseur.

Avantageusement, la surface en contact avec l'air au niveau de la zone d'échange du gel diffuseur avec l'air est contrôlée par des moyens d'obturations réglables des ouvertures. Ces moyens d'obturation réglables permettent par le contrôle de la zone d'échange du gel diffuseur avec l'air, de réduire ou d'augmenter la vitesse de diffusion de la composition sémiochimique dans l'environnement en fonction du besoin de l'animal et/ou de la taille de la pièce. Le diamètre des ouvertures requis et le nombre d'ouvertures nécessaire pour obtenir une diffusion adéquate peuvent être déterminés expérimentalement.

La zone d'échange du gel diffuseur avec l'air est avantageusement recouverte par un couvercle amovible étanche qui permet de pouvoir stopper la diffusion dans l'air de la composition sémiochimique lorsqu'il est en place. Ainsi, la diffusion ou l'évaporation naturelle de la composition sémiochimique peut être très fortement réduite, voire être stoppée, ce qui est particulièrement utile car économique et écologique, lorsque le dispositif diffuseur n'est plus à proximité du chien ou du chat (lorsque le chien ou le chat est en promenade par exemple, ou lorsqu'il n'est pas requis de lui administrer la composition apaisante).

Dans un mode de réalisation particulier reprenant les caractéristiques décrites ci-dessus, l'invention concerne un récipient ayant une paroi inférieure, une paroi latérale, une paroi supérieure ou zone d'échange du gel diffuseur avec l'air ayant une pluralité d'ouvertures et un couvercle ayant une pluralité d'ouvertures capable de pivoter par rapport à la paroi supérieure afin d'aligner ou de désaligner la pluralité d'ouvertures de la paroi supérieure et la pluralité d'ouvertures du couvercle. Dans ce mode de réalisation, la paroi supérieure et le couvercle définissent un système de contrôle de diffusion de la composition sémiochimique en augmentant, en réduisant ou en supprimant l'échange du gel diffuseur avec l'air avec l'environnement de l'animal. Un tel système de contrôle de la surface d'échange entre le gel diffuseur et l'air permet d'adapter le relargage à la dimension de la pièce dans laquelle se trouve l'animal et d'augmenter la durée de vie du dispositif diffuseur en assurant la stabilité de la composition sémiochimique que renferme le gel diffuseur lorsque le dispositif diffuseur est fermé de manière étanche quand son usage est inutile.

Selon un autre mode de réalisation particulier, le récipient dispose d'un double couvercle, le premier couvercle comportant des orifices et étant fixé directement sur le récipient, le second couvercle se fixant au-dessus du premier et permettant la fermeture étanche des orifices du premier couvercle ; pour la mise en oeuvre du dispositif de ce mode de réalisation, l'utilisateur pourra, lorsqu'il aura choisi le lieu adapté où positionner le dispositif, ouvrir le second couvercle, la composition sémiochimique pourra alors diffuser passivement du gel diffuseur à l'air par les orifices du premier couvercle.

Pour faciliter le transport et/ou la conservation du gel diffuseur conditionné dans un récipient, ce récipient peut comprendre un moyen permettant une fermeture étanche, telle qu'un couvercle et/ou un opercule. Pour faciliter le transport, une poignée pourra équiper le dispositif diffuseur. De façon optionnelle, le dispositif peut encore comporter des moyens de fixation tels qu'un crochet, une bande adhésive, etc...

La présente invention concerne également un procédé de préparation d'un gel diffuseur tel que défini précédemment, comprenant les étapes de :
1) chauffage, à une température suffisante et pendant un temps suffisant pour solubiliser le gélifiant polysaccharidique, d'un mélange comprenant :
   - un gélifiant polysaccharidique,
   - une composition sémiochimique,
   - un agent solubilisant de ladite composition sémiochimique,
   - un acide organique ayant un pKa supérieur à 3,
   - optionnellement, un conservateur,
   - optionnellement, un colorant,
   - optionnellement, un agent amérisant,
2) refroidissement de la solution obtenue à l'étape 1) de manière à obtenir un gel diffuseur tel que défini précédemment.

Le gel diffuseur selon l'invention est avantageusement préparé en mélangeant la composition sémiochimique et l'agent solubilisant de la composition sémiochimique de manière à obtenir un premier pré-mélange.

L'ensemble des étapes de mélange et/ou de chauffage est réalisé de préférence sous agitation.

Un deuxième pré-mélange comprenant l'eau, l'acide organique ayant un pKa supérieur à 3, le colorant et l'agent amérisant est préparé. Avantageusement, le deuxième pré-mélange est préparé en mélangeant d'abord l'eau et l'acide organique ayant un pKa supérieur à 3, optionnellement le colorant et l'agent amérisant, et en chauffant à une température suffisante et pendant un temps suffisant de manière à obtenir une première solution dans laquelle les ingrédients sont entièrement solubilisés. Typiquement, cette première solution est obtenue par chauffage à une température comprise entre 50 et 100 °C, notamment à une température d'environ 70 °C.

A cette première solution est ensuite ajouté le gélifiant polysaccharidique et le mélange est chauffé à une température suffisante et pendant un temps suffisant de manière à obtenir une deuxième solution dans laquelle le gélifiant polysaccharidique est entièrement dissous. Les gélifiants polysaccharidiques pouvant se dégrader lorsqu'ils sont chauffés à une température trop élevée et/ou pendant un temps trop long, il est alors nécessaire de chauffer le mélange à une température appropriée et pendant une durée approprié.

La température et le temps de chauffage peuvent être déterminés par des essais de routine. Typiquement, lorsque le gélifiant polysaccharidique est un carraghénane, la solution est chauffée à une température de l'ordre de 70°C pendant une durée d'au moins 1h. Un simple contrôle visuel permet de vérifier que le gélifiant polysaccharidique est totalement dissous.

Lorsque le gélifiant polysaccharidique est entièrement dissous, le premier pré-mélange est ajouté à la première solution.

Le premier pré-mélange peut être ajouté à une température identique à celle à laquelle la première solution est préparée ou à une température différente. Dans un mode de réalisation avantageux, le premier pré-mélange est ajouté à une température inférieure à la température à laquelle la première solution est préparée mais à laquelle le gélifiant polysaccharidique est toujours solubilisé. Lorsqu'un conservateur est ajouté, il est ajouté à la première solution, avantageusement après le premier pré-mélange.

Après l'addition du premier pré-mélange et éventuellement du conservateur à la première solution, on obtient une deuxième solution comprenant :
- un gélifiant polysaccharidique,
- une composition sémiochimique,
- un agent solubilisant de ladite composition sémiochimique,
- un acide organique ayant un pKa supérieur à 3,
- optionnellement, un conservateur,
- optionnellement, un colorant,
- optionnellement, un agent amérisant.

Ladite deuxième solution est maintenue à une température suffisante pour permettre la formation d'une solution homogène qui peut être répartie dans des dispositifs diffuseurs, tels que décrits ci-dessus. En refroidissant à une température de l'ordre de 15 à 25°C (température ambiante), la deuxième solution solidifie pour former un gel diffuseur tel que décrit ci-dessus. Alternativement, la solution homogène peut être refroidie directement et le bloc de gel formé après le refroidissement à température ambiante peut être divisé en autant de parties que désiré.

Dans un mode de réalisation avantageux, la présente invention concerne un procédé de préparation d'un gel diffuseur tel que défini précédemment, comprenant les étapes de :
1) mélange d'une composition sémiochimique et d'un agent solubilisant de la composition sémiochimique de manière à obtenir un premier pré-mélange,
2) mélange d'eau, d'un acide organique ayant un pKa supérieur à 3, optionnellement d'un colorant et d'un agent amérisant, et chauffage à une température suffisante et pendant un temps suffisant de manière à obtenir une première solution,
3) ajout d'un gélifiant polysaccharidique à la première solution et chauffage, à une température suffisante et pendant un temps suffisant, de manière à obtenir un deuxième pré-mélange dans lequel le gélifiant polysaccharidique est entièrement dissous,
4) ajout du premier pré-mélange obtenu à l'étape 1), et éventuellement d'un conservateur, à la première solution obtenue à l'étape 3), et chauffage du mélange de manière à obtenir une solution homogène,
5) refroidissement de la solution homogène obtenue à l'étape 4 de manière à obtenir un gel diffuseur tel que défini précédemment.

Dans un mode de réalisation préféré, la présente invention concerne un procédé de préparation d'un gel diffuseur tel que défini précédemment, comprenant les étapes de :
1) mélange d'une composition sémiochimique et d'isopropanol, de manière à obtenir un premier pré-mélange,
2) mélange d'eau, d'acide citrique, d'un colorant et d'un agent amérisant, et chauffage à une température suffisante et pendant un temps suffisant de manière à obtenir une première solution,
3) ajout d'un carraghénane à la première solution et chauffage, à une température suffisante et pendant un temps suffisant, de manière à obtenir une deuxième solution dans lequel le carraghénane est entièrement dissous,
4) ajout du premier pré-mélange obtenu à l'étape 1), et éventuellement du conservateur, à la deuxième solution obtenue à l'étape 3), et chauffage du mélange de manière à obtenir une solution homogène,
5) refroidissement de la solution homogène obtenue à l'étape 4) de manière à obtenir un gel diffuseur tel que défini précédemment.

La présente invention se rapporte encore à une méthode non thérapeutique pour apaiser un animal, en particulier pour :
- assurer le bien-être d'un animal, dans son environnement,
- aider un animal à gérer les situations de stress et/ou d'anxiété,
- réduire le stress associé à la séparation chez un animal,
- réduire le stress associé au sevrage chez un animal,
- réduire le stress associé à la solitude chez un animal,
- réduire le stress chez un animal avant et/ou pendant un transport,
- réduire le stress associé aux bruits tels que les orages ou les feux d'artifice,
- réduire le stress associé à une pathologie, un traitement médical, la visite chez un vétérinaire pouvant nécessiter une osculation, voire une intervention chirurgicale,
- réduire l'agressivité d'un animal,
- améliorer le comportement d'un animal, en particulier d'un animal agressif ou d'un animal peureux,
- produire un effet relaxant sur l'animal,
- aider un animal à s'adapter à une situation nouvelle par exemple au moment de l'adoption, lors de l'arrivée d'un nouvel animal ou d'un enfant dans son environnement, lors d'un déménagement,
- réduire le marquage urinaire associé au stress chez un animal,
- réduire la malpropreté et les souillures d'un animal,
- améliorer les conditions de familiarisation d'un animal avec ses congénères,
- empêcher les griffades ou la destruction du territoire,
- diminuer les manifestations sonores telles que pleurs, aboiements,
- engendrer un comportement moins agressif, plus détendu et plus affectueux notamment avec leurs maitres chez un animal,
comprenant la mise en oeuvre d'un dispositif diffuseur selon l'invention dans l'environnement dudit animal.

La présente invention se rapporte encore à une composition sémiochimique telle que définie ci-dessus pour prévenir les manifestations de stress et/ou réduire l'anxiété chez un animal de compagnie pendant une durée supérieure à 1 mois, plus avantageusement supérieure à 2 mois, de préférence pendant une durée d'environ 3 mois, caractérisée en ce que la composition sémiochimique est présente dans un gel diffuseur selon l'invention.

En particulier, elle se rapporte à une composition sémiochimique telle que définie ci-dessus pour son utilisation pour apaiser un animal, en particulier pour :
- assurer le bien-être d'un animal, dans son environnement,
- aider un animal à gérer les situations de stress et/ou d'anxiété,
- réduire le stress associé à la séparation chez un animal,
- réduire le stress associé au sevrage chez un animal,
- réduire le stress associé à la solitude chez un animal,
- réduire le stress chez un animal avant et/ou pendant un transport,
- réduire le stress associé aux bruits tels que les orages ou les feux d'artifice,
- réduire le stress associé à une pathologie, un traitement médical, la visite chez un vétérinaire pouvant nécessiter une auscultation, voire une intervention chirurgicale,
- réduire l'agressivité d'un animal,
- améliorer le comportement d'un animal, en particulier d'un animal agressif ou d'un animal peureux,
- produire un effet relaxant sur l'animal,
- aider un animal à s'adapter à une situation nouvelle par exemple au moment de l'adoption, lors de l'arrivée d'un nouvel animal ou d'un enfant dans son environnement, lors d'un déménagement,
- réduire le marquage urinaire associé au stress chez un animal,
- réduire la malpropreté et les souillures d'un animal,
- améliorer les conditions de familiarisation d'un animal avec ses congénères,
- empêcher les griffades ou la destruction du territoire,
- diminuer les manifestations sonores telles que pleurs, aboiements,
- engendrer un comportement moins agressif, plus détendu et plus affectueux notamment avec leurs maitres chez un animal,
- faciliter le dressage et/ou l'éducation d'un animal,
la composition sémiochimique étant comprise dans un gel diffuseur tel que défini précédemment comprenant :
- une matrice gélifiée,
- une composition sémiochimique,
- un agent solubilisant de ladite composition sémiochimique dans ladite matrice gélifiée,
- un acide organique ayant un pKa supérieur à 3,
- optionnellement, un conservateur,
- optionnellement, un colorant,
- optionnellement, un agent amérisant.

### FIGURES

[Fig.1] : graphe représentant le profil de diffusion de la composition sémiochimique formulée dans le gel diffuseur de l'exemple 1.
[Fig. 2] et [Fig. 3] : graphes comparant l'évolution de comportements (figure 2) et de signes (figure 3) de chiens mis en présence d'un dispositif diffuseur selon l'invention ou du diffuseur Adaptil^{®} Calm (voir exemple 4). La courbe en pointillés représente les résultats obtenus avec le diffuseur Adaptil^{®} Calm et la courbe pleine les résultats obtenus avec le dispositif diffuseur selon l'invention.

### EXEMPLES

### Exemple 1 : préparation d'un gel diffuseur selon l'invention

La fraction phéromonale et l'isopropanol sont mélangés sous agitation dans un réacteur. Dans un réacteur séparé, l'acide citrique, le benzoate de dénathonium, le colorant *Food yellow* 3 et l'eau sont ajoutés et chauffés sous agitation à 70 °C. Lorsque tous les composants sont dissous, le carraghénane est ajouté et le mélange est agité jusqu'à l'obtention d'une solution homogène à 70 °C (typiquement au moins 1h). Le mélange est refroidi à une température de 50 °C (afin de ne pas endommager les esters présents dans la fraction phéromonale). La fraction phéromonale dissoute dans l'isopropanol est ajoutée au mélange refroidi, suivie de la la benzoisothiazolinone. Le mélange final est agité à 50 °C pendant environ 1 heure puis réparti dans des dispositifs diffuseurs.

Les dispositifs diffuseurs sont laissés à température ambiante jusqu'à formation d'un gel. Ils sont ensuite scellés.

**[Table 1]**

| Dénomination chimique | Fonction | %m/m |
|---|---|---|
| Laurate de méthyle | Fraction phéromonale (total 6%) | 0,118% |
| Myristate de méthyle | | 0,287% |
| Palmitate de méthyle | | 1,276% |
| Stéarate de méthyle | | 0,927% |
| Oléate de méthyle | | 2,113% |
| Linoléate de méthyle | | 1,280% |
| Propan-2-ol | Solubilisant actifs & Aide à l'évaporation | 4,000% |
| Benzoisothiazolinone | Conservateur | 0,180% |
| Acide citrique | Ajusteur de pH | 0,050% |
| Carraghénanes | Gélifiiant | 1,500% |
| Benzoate de dénatonium | Amérisant | 0,080% |
| *Food yellow 3* | Colorant | 0,0002% |
| eau | | 88,1888% |

### Exemple 2 - évaluation du profil de diffusion de la composition sémiochimique à partir du gel diffuseur de l'exemple 1 : Etude conduite en conditions contrôlées de température, d'humidité et de ventilation (en moyenne 22,5°C / 54% HR, pièce ventilée sur la période).

Un nombre de dispositifs diffuseurs suffisant pour réaliser des prélèvements à 7 jours d'intervalle pendant 63 jours est placé à T0 dans une pièce ventilée en conditions contrôlées de température, d'humidité et de ventilation (en moyenne 22,5°C / 54% HR, pièce avec airflow sur la période). Un dispositif est prélevé à J 7, 17, 21, 28, 35, 42, 52, 56 et 63 et son contenu est analysé par chromatographie en phase gazeuse (CPG). Les échantillons sont préparés de la manière suivante :
1. le gel est divisé en morceaux d'environ 3 g chacun,
2. on ajoute à l'échantillon de 3 g 60 mL de NaOH 30% jusqu'à dissolution complète,
3. lorsque la totalité du gel est dissous dans le NaOH, on ajoute 30 mL d'acétate d'éthyle, on agite le mélange et on laisse décanter,
4. un échantillon de la phase organique est prélevé et analysé par chromatographie en phase gazeuse (détection par FID).

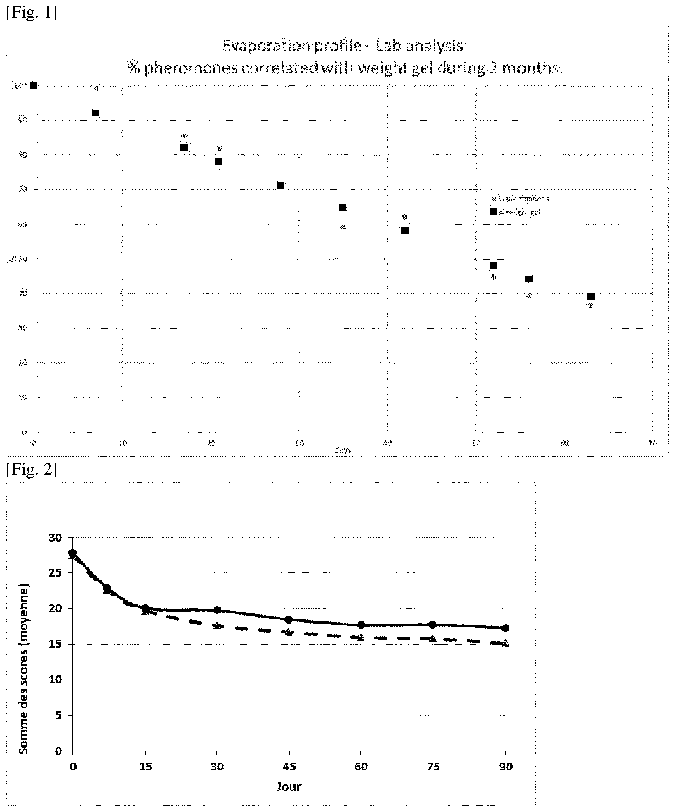

Le profil de diffusion de la composition sémiochimique formulée dans le gel diffuseur de l'exemple 1 est représenté sur le graphe de la figure 1.

Ces résultats montrent que la composition sémiochimique selon l'invention est libérée de manière continue et régulière dans l'environnement pendant plus de 60 jours lorsque le gel diffuseur est en contact avec l'air.

### Exemple 3 - étude de la stabilité de la composition sémiochimique dans le gel diffuseur de l'exemple 1

Des lots du gel diffuseur de l'exemple 1 conditionné dans un dispositif diffuseur fermé ont été conservés dans différentes conditions de stockage :
▪ 3 semaines à 54°C (conditions CIPAC MT46.3 « Accelerated Storage Procedure ») ;
▪ 6 mois à 40°C.

Le dosage des phéromones a ensuite été réalisé par chromatographie.

Les résultats montrent que la teneur en phéromones reste constante pendant toute la durée de stockage et que le gel diffuseur selon l'invention préserve bien de la composition sémiochimique et assure sa conservation lorsque le dispositif est étanche à l'air.

### Exemple 4 - étude de perception d'efficacité

Cette étude a été réalisée au domicile de propriétaires de chiens en France (93 propriétaires) et en Grande Bretagne (103 propriétaires) ; les foyers retenus pour cette étude ne comportent qu'un chien âgé de plus de 4 mois vivant essentiellement en intérieur/appartement (sortant moins de 4h par jour) et présentant des troubles du comportement mineur (type anxiété de séparation...), ils n'ont pas de chat. Les chiens n'ont pas été traités pour des troubles du comportement avant et pendant l'étude.

2 produits ont été évalués pendant 3 mois par des groupes de sujets indépendants :
- le dispositif diffuseur contenant le gel diffuseur de l'exemple 1 (un seul dispositif utilisé pendant les 3 mois) ; et
- le produit commercial Adaptil^{®} Calm (1 starter kit comprenant le diffuseur électrique et une recharge + 2 recharges, chaque recharge étant utilisée pendant 1 mois).

L'évolution de 17 comportements :
Pleurniche, geint souvent
Aboie de manière excessive ou dès qu'il entend un bruit
Grogne sur les gens et/ou sur les autres animaux
Détruit/déchiquette les objets, meubles, chaussures, jouets, poubelles...
Fait souvent ses besoins dans un lieu inhabituel/inapproprié
Mange ses excréments
Fait pipi dès qu'il a une émotion (content, peur,...)
Tremble beaucoup
Se cache souvent ou essaye de se cacher
Porte la tête et/ou le cou bas, queue entre les jambes
Sursaute au moindre bruit
A peur en voiture
A peur de l'orage/des feux d'artifices
A peur des autres animaux
N'aime pas être seul (pleurniche, aboie, ne mange pas en notre absence...)
Reste prostré (immobilité, peu d'activités, de jeux)
Se lèche les pattes sans raison particulière (en l'absence de plaie, blessure, parasites...)
et 11 signes :
   Bâillements,
   Tremblements,
   Cris/aboiements,
   Léchage des pattes,
   Agitation,
   Position de la queue basse,
   Oreilles vers l'arrière, Position du corps recroquevillée/accroupie,
   Se cache / essaie de se cacher,
ont été évalués par les propriétaires de chiens dans l'environnement desquels l'un ou l'autre des diffuseurs a été installé.

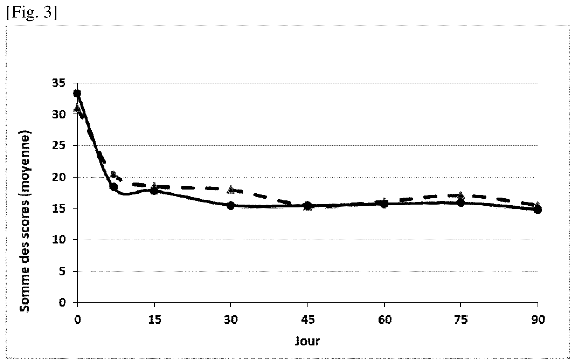

Les figures 2 et 3 présentent les résultats obtenus pour l'ensemble des animaux. Ces résultats montrent que le diffuseur selon l'invention présente une efficacité pendant 3 mois équivalente à celle d'un diffuseur électrique dont la recharge a été remplacée chaque mois.

## Revendications

1. Gel diffuseur passif de compositions sémiochimiques pour chiens et/ou pour chats comprenant :
- une matrice gélifiée polysaccharide,
- une composition sémiochimique apaisante pour chiens et/ou pour chats comprenant un mélange de dérivés volatils d'acide gras, ledit dérivé volatil d'acide gras étant un ester éthylique ou méthylique d'acide gras,
- un agent solubilisant de ladite composition sémiochimique dans ladite matrice gélifiée,
- un acide organique ayant un pKa supérieur à 3,
- optionnellement, un conservateur -optionnellement, un colorant,
- optionnellement, un agent amérisant.

2. Gel diffuseur selon la revendication 1, **caractérisé en ce que** la matrice gélifiée est un gel aqueux de carraghénane.

3. Gel diffuseur selon la revendication 1 ou la revendication 2, **caractérisé en ce que** l'agent solubilisant est un alcool en C1 à C4.

4. Gel diffuseur selon l'une des revendications 1 à 3, **caractérisé en ce que** l'acide organique ayant un pKa supérieur à 3 est l'acide citrique.

5. Gel diffuseur selon l'une des revendications 1 à 4, **caractérisé en ce que** l'acide gras est choisi dans le groupe constitué de l'acide oléique, l'acide palmitique, l'acide azélaïque, l'acide pimélique, l'acide caprique, l'acide laurique, l'acide myristique, l'acide palmitoléique, l'acide linoléique, l'acide stéarique, l'acide arachidonique, l'acide n-butyrique, l'acide isobutyrique, l'acide α-méthylbutyrique, l'acide caproïque, l'acide pivalique, l'acide γ-linoléique, l'acide eicosapentanoïque, l'acide pentadécanoïque, l'acide tridécanoïque, ou l'acide docosahexanoïque, avantageusement choisi dans le groupe constitué de l'acide oléique, l'acide palmitique, l'acide laurique, l'acide myristique, l'acide linoléique, l'acide stéarique, et l'acide pentadécanoïque.

6. Gel diffuseur selon l'une des revendications 1 à 5, dans lequel la composition sémiochimique est un mélange d'esters d'acides gras comprenant entre 32% et 38% d'oléate de méthyle, entre 1 et 3% de laurate de méthyle, entre 13 et 16% de stéarate de méthyle, entre 18 et 24% de linoléate de méthyle, entre 3 et 7% de myristate de méthyle et entre 18 et 24% de palmitate de méthyle.

7. Gel diffuseur selon l'une des revendications 1 à 6, dans lequel la composition sémiochimique est comprise entre 1% et 10% en masse de la masse totale du gel diffuseur.

8. Gel diffuseur selon l'une quelconque des revendications précédentes **caractérisé en ce qu'**il a la composition suivante :
- une matrice gélifiée comprenant un gel aqueux de carraghénane ;
- de 1 à 10% d'une composition sémiochimique, ladite composition sémiochimique étant un mélange d'esters d'acides gras comprenant entre 32% et 38% d'oléate de méthyle, entre 1 et 3% de laurate de méthyle, entre 13 et 16% de stéarate de méthyle, entre 18 et 24% de linoléate de méthyle, entre 3 et 7% de myristate de méthyle et entre 18 et 24% de palmitate de méthyle, de préférence environ 6% ;
- de 2 à 10% d'un agent solubilisant de ladite composition sémiochimique dans ladite matrice gélifiée choisi parmi les alcools en C1 à C4 ; -entre 0,1 et 1% d'acide citrique ;
- de 0,1 à 0,5% d'un conservateur de la famille des isothiazolinones ;
- un colorant ;
- du benzoate de dénatonium à titre d'agent amérisant.

9. Dispositif diffuseur portable pour le relargage contrôlé dans l'environnement d'un chat ou d'un chien d'une composition sémiochimique à partir d'un gel diffuseur selon l'une quelconque des revendications 1 à 8 **caractérisé en ce qu'**il est constitué d'un récipient comportant une zone d'échange du gel diffuseur avec l'air.

10. Dispositif diffuseur selon la revendication 9 **caractérisé en ce que** la zone d'échange du gel diffuseur avec l'air est constituée de une ou une pluralité d'ouvertures par lesquelles la composition sémiochimique peut diffuser.

11. Dispositif diffuseur selon l'une quelconque des revendications 9 et 10 **caractérisé en ce que** la zone d'échange du gel diffuseur avec l'air est recouverte par un couvercle amovible étanche.

12. Dispositif diffuseur selon l'une quelconque des revendications 9 à 11 **caractérisé en ce que** la surface en contact avec l'air au niveau de la zone d'échange du gel diffuseur avec l'air est contrôlée par des moyens d'obturations réglables des ouvertures.

13. Gel diffuseur selon l'une quelconque des revendications 1 à 8 pour son utilisation pour:
- assurer le bien-être d'un animal, dans son environnement,
- aider un animal à gérer les situations de stress et/ou d'anxiété,
- réduire le stress associé à la séparation chez un animal,
- réduire le stress associé au sevrage chez un animal,
- réduire le stress associé à la solitude chez un animal,
- réduire le stress chez un animal avant et/ou pendant un transport,
- réduire le stress associé aux bruits tels que les orages ou les feux d'artifice,
- réduire le stress associé à une pathologie, un traitement médical, la visite chez un vétérinaire pouvant nécessiter une osculation, voire une intervention chirurgicale,
- réduire l'agressivité d'un animal, -améliorer le comportement d'un animal, en particulier d'un animal agressif ou d'un animal peureux,
- produire un effet relaxant sur l'animal,
- aider un animal à s'adapter à une situation nouvelle par exemple au moment de l'adoption, lors de l'arrivée d'un nouvel animal ou d'un enfant dans son environnement, lors d'un déménagement,
- réduire le marquage urinaire associé au stress chez un animal,
- réduire la malpropreté et les souillures d'un animal,
- améliorer les conditions de familiarisation d'un animal avec ses congénères,
- empêcher les griffades ou la destruction du territoire, -diminuer les manifestations sonores telles que pleurs, aboiements, ou
- engendrer un comportement moins agressif, plus détendu et plus affectueux notamment avec leurs maitres chez un animal,
avantageusement pendant une durée supérieure à 1 mois, plus avantageusement supérieure à 2 mois, de préférence pendant une durée d'environ 3 mois, l'animal étant un chien ou un chat.

14. Gel diffuseur selon l'une quelconque des revendications 1 à 8, pour son utilisation dans le traitement du stress, de l'anxiété et/ou d'un comportement agressif chez un chat ou chez un chien.

## Patentansprüche

1. Passives Diffusionsgel für semiochemische Zusammensetzungen für Hunde und/oder für Katzen, umfassend:
- eine gelierte Polysaccharidmatrix,
- eine beruhigende semiochemische Zusammensetzung für Hunde und/oder für Katzen, umfassend eine Mischung aus flüchtigen Fettsäurederivaten, wobei das flüchtige Fettsäurederivat ein Fettsäureethyl- oder -methylester ist,
- ein Lösungsmittel der semiochimischen Zusammensetzung in der gelierten Matrix,
- eine organische Säure, die einen pKa-Wert größer als 3 aufweist,
- wahlweise ein Konservierungsmittel -wahlweise ein Farbmittel,
- wahlweise ein Bitterstoffmittel.

2. Diffusionsgel nach Anspruch 1, **dadurch gekennzeichnet, dass** die gelierte Matrix ein wässriges Carrageengel ist.

3. Diffusionsgel nach Anspruch 1 oder Anspruch 2, **dadurch gekennzeichnet, dass** das Lösungsmittel ein C1- bis C4-Alkohol ist.

4. Diffusionsgel nach einem der Ansprüche 1 bis 3, **dadurch gekennzeichnet, dass** die organische Säure, die einen pKa-Wert größer als 3 aufweist, Zitronensäure ist.

5. Diffusionsgel nach einem der Ansprüche 1 bis 4, **dadurch gekennzeichnet, dass** die Fettsäure aus der Gruppe ausgewählt ist, die aus Ölsäure, Palmitinsäure, Azelainsäure, Pimelinsäure, Caprinsäure, Laurinsäure, Myristinsäure, Palmitoleinsäure, Linolsäure, Stearinsäure, Arachidonsäure, n-Buttersäure, Isobuttersäure, α-Methylbuttersäure, Caproinsäure, Pivalinsäure, γ-Linolsäure, Eicosapentaensäure, Pentadecansäure, Tridecansäure oder Docosahexaensäure besteht, vorzugsweise ausgewählt aus der Gruppe, die aus Ölsäure, Palmitinsäure, Laurinsäure, Myristinsäure, Linolsäure, Stearinsäure und Pentadecansäure besteht.

6. Diffusionsgel nach einem der Ansprüche 1 bis 5, wobei die semiochemische Zusammensetzung eine Mischung aus Fettsäureestern ist, die zwischen 32% und 38% Methyloleat, zwischen 1 und 3% Methyllaurat, zwischen 13 und 16% Methylstearat, zwischen 18 und 24% Methyllinoleat, zwischen 3 und 7% Methylmyristat und zwischen 18 und 24% Methylpalmitat umfasst.

7. Diffusionsgel nach einem der Ansprüche 1 bis 6, wobei die semiochemische Zusammensetzung zwischen 1% und 10% Masse der Gesamtmasse des Diffusionsgels umfasst.

8. Diffusionsgel nach einem der vorstehenden Ansprüche, **dadurch gekennzeichnet, dass** es die folgende Zusammensetzung aufweist:
- eine gelierte Matrix, die ein wässriges Carrageengel umfasst;
- 1 bis 10% einer semiochemischen Zusammensetzung, wobei die semiochemische Zusammensetzung eine Mischung aus Fettsäureestern ist, die zwischen 32% und 38% Methyloleat, zwischen 1 und 3% Methyllaurat, zwischen 13 und 16% Methylstearat, zwischen 18 und 24% Methyllinoleat, zwischen 3 und 7% Methylmyristat und zwischen 18 und 24% Methylpalmitat umfasst, vorzugsweise etwa 6%;
- 2 bis 10% eines Lösungsmittels der semiochemischen Zusammensetzung in der gelierten Matrix, das aus den C1- bis C4-Alkoholen ausgewählt ist; - zwischen 0,1 und 1% Zitronensäure;
- 0,1 bis 0,5% eines Konservierungsmittels der Familie der Isothiazolinone;
- einen Farbstoff;
- Denatoniumbenzoat als Bitterstoffmittel.

9. Tragbare Diffusionsvorrichtung für die gesteuerte Abgabe einer semiochemischen Zusammensetzung auf Basis eines Diffusionsgels nach einem der Ansprüche 1 bis 8 in der Umgebung einer Katze oder eines Hundes, **dadurch gekennzeichnet, dass** sie aus einem Behälter besteht, der einen Austauschbereich des Diffusionsgels mit der Luft umfasst.

10. Tragbare Diffusionsvorrichtung nach Anspruch 9, **dadurch gekennzeichnet, dass** der Austauschbereich des Diffusionsgels mit der Luft aus einer oder einer Vielzahl von Öffnungen besteht, durch die die semiochemische Zusammensetzung diffundieren kann.

11. Tragbare Diffusionsvorrichtung nach einem der Ansprüche 9 und 10, **dadurch gekennzeichnet, dass** der Austauschbereich des Diffusionsgels mit der Luft von einem dichten abnehmbaren Deckel bedeckt ist.

12. Tragbare Diffusionsvorrichtung nach einem der Ansprüche 9 bis 11, **dadurch gekennzeichnet, dass** die Oberfläche im Kontakt mit der Luft am Austauschbereich des Diffusionsgels mit der Luft durch einstellbare Verschlussmittel der Öffnungen gesteuert wird.

13. Diffusionsgel einem der Ansprüche 1 bis 8, für dessen Verwendung zum:
- Sicherstellen des Wohlergehens eines Tiers in seiner Umgebung,
- Unterstützen eines Tiers dabei, mit Stress- und/oder Angstsituationen umzugehen,
- Verringern des mit der Trennung verbundenen Stresses bei einem Tier,
- Verringern des mit dem Absetzen verbundenen Stresses bei einem Tier,
- Verringern des mit dem Allseinsein verbundenen Stresses bei einem Tier,
- Verringern des Stresses bei einem Tier vor und/oder während eines Transports,
- Verringern des mit dem Lärm, wie etwa Gewitter oder Feuerwerk, verbundenen Stresses bei einem Tier,
- Verringern des mit einer Pathologie, einer medizinischen Behandlung, dem Besuch bei einem Tierarzt, der eine Untersuchung, sogar einen chirurgischen Eingriff erfordern kann, verbundenen Stresses bei einem Tier,
- Verringern der Aggressivität bei einem Tier, -Verbessern des Verhaltens eines Tiers, insbesondere eines aggressiven Tiers oder eines ängstlichen Tiers,
- Erzeugen einer entspannenden Wirkung auf das Tier,
- Unterstützen eines Tiers dabei, sich an eine neue Situation anzupassen, zum Beispiel zum Zeitpunkt der Adoption, bei der Ankunft eines neuen Tiers oder eines Kindes in seiner Umgebung, bei einem Umzug,
- Verringern der mit Stress verbundenen Harnmarkierung bei einem Tier,
- Verringern der Unreinheit und der Beschmutzung bei einem Tier,
- Verbessern der Vertrautmachungsbedingungen eines Tiers mit seinen Artgenossen,
- Verhindern des Kratzens oder der Zerstörung des Reviers, -Abschwächen der Lautäußerungen, wie etwa Weinen, Bellen, oder
- Hervorbringen eines weniger aggressiven, entspannteren und liebevolleren Verhaltens bei einem Tier, insbesondere gegenüber seinen Haltern,
vorteilhafterweise während einer Dauer von mehr als 1 Monat, noch vorteilhafter mehr als 2 Monaten, vorzugsweise während einer Dauer von etwa 3 Monaten, wobei das Tier ein Hund oder eine Katze ist.

14. Diffusionsgel nach einem Ansprüche 1 bis 8, für dessen Verwendung bei der Behandlung von Stress, Ängstlichkeit und oder eines aggressiven Verhaltens bei einer Katze oder bei einem Hund.

## Claims

1. A passive diffusing gel for semiochemical compositions for dogs and/or cats comprising:
- a polysaccharide gel matrix,
- a soothing semiochemical composition for dogs and/or cats comprising a mixture of volatile fatty acid derivatives, said volatile fatty acid derivative being a fatty acid ethyl or methyl ester,
- a solubilising agent of said semiochemical composition in said gel matrix,
- an organic acid with a pKa greater than 3,
- optionally, a preservative -optionally, a colouring agent,
- optionally, a bittering agent.

2. The diffusing gel according to claim 1, **characterised in that** the gel matrix is an aqueous gel of carrageenan.

3. The diffusing gel according to claim 1 or claim 2, **characterised in that** the solubilising agent is a C1 to C4 alcohol.

4. The diffusing gel according to one of claims 1 to 3, **characterized in that** the organic acid having a pKa greater than 3 is the citric acid.

5. The diffusing gel according to one of claims 1 to 4, **characterized in that** the fatty acid is chosen from the group consisting of oleic acid, palmitic acid, azelaic acid, pimelic acid, capric acid, lauric acid, myristic acid, palmitoleic acid, linoleic acid, stearic acid, arachidonic acid, n-butyric acid, isobutyric acid, α-methylbutyric acid, caproic acid, pivalic acid, γ-linoleic acid, eicosapentanoic acid, pentadecanoic acid, tridecanoic acid, or docosahexanoic acid, advantageously chosen from the group consisting of oleic acid, palmitic acid, lauric acid, myristic acid, linoleic acid, stearic acid and pentadecanoic acid.

6. The diffusing gel according to one of claims 1 to 5, wherein the semiochemical composition is a mixture of fatty acid esters comprising between 32% and 38% of methyl oleate, between 1 and 3% of methyl laurate, between 13 and 16% of methyl stearate, between 18 and 24% of methyl linoleate, between 3 and 7% of methyl myristate and between 18 and 24% of methyl palmitate.

7. The diffusing gel according to one of claims 1 to 6, wherein the semiochemical composition is between 1% and 10% by mass of the total mass of the diffusing gel.

8. The diffusing gel according to any one of the preceding claims, **characterized in that** it has the following composition:
- a gel matrix comprising an aqueous gel of carrageenan;
- from 1 to 10% of a semiochemical composition, said semiochemical composition being a mixture of fatty acid esters comprising between 32% and 38% of methyl oleate, between 1 and 3% of methyl laurate, between 13 and 16% of methyl stearate, between 18 and 24% of methyl linoleate, between 3 and 7% of methyl myristate and between 18 and 24% of methyl palmitate, preferably about 6%;
- from 2 to 10% of a solubilising agent of said semiochemical composition in said gel matrix, chosen from C1 to C4 alcohols; -between 0.1 and 1% citric acid;
- 0.1 to 0.5% of a preservative from the isothiazolinone family;
- a colouring agent;
- the denatonium benzoate as a bittering agent.

9. A portable diffusing device for the controlled release into the environment of a cat or dog of a semiochemical composition from a diffusing gel according to any one of claims 1 to 8, **characterized in that** it consists of a container comprising a zone for exchanging the diffusing gel with the air.

10. The diffusing device according to claim 9, **characterized in that** the exchange zone of the diffusing gel with the air consists of one or a plurality of openings through which the semiochemical composition can diffuse.

11. The diffusing device according to any one of claims 9 and 10, **characterised in that** the exchange zone of the diffusing gel with the air is covered by a tight removable cover.

12. The diffusing device according to any one of claims 9 to 11, **characterised in that** the surface in contact with the air at the level of the exchange zone of the diffusing gel with the air is controlled by adjustable means for closing the openings.

13. The diffusing gel according to any one of claims 1 to 8 for use in:
- ensuring the well-being of an animal in its environment,
- helping an animal to manage stressful and/or anxious situations,
- reducing the stress associated with separation in an animal,
- reducing the stress associated with weaning an animal,
- reducing the stress associated with loneliness in an animal,
- reducing the stress in an animal before and/or during transport,
- reducing the stress associated with noises such as thunderstorms or fireworks,
- reducing the stress associated with a pathology, medical treatment or a visit to a veterinary that may require osculation or even surgical procedure,
- reducing an animal's aggressiveness, - improving an animal's behaviour, particularly that of an aggressive or timid animal,
- producing a relaxing effect on the animal,
- helping an animal to adapt to a new situation, for example when it is adopted, when a new animal or child arrives in its environment, during a move,
- reducing the urinary marking associated with stress in an animal,
- reducing the uncleanliness and soiling of an animal,
- improving the conditions for familiarising an animal with its fellow creatures,
- preventing scratching or destruction of the territory, - reducing noises such as crying, barking, or
- generating a less aggressive, more relaxed and more affectionate behaviour in animals, particularly with their owners,
advantageously for more than 1 month, more advantageously for more than 2 months, preferably for about 3 months, the animal being a dog or cat.

14. The diffusing gel according to any one of claims 1 to 8, for use in the treatment of stress, anxiety and/or aggressive behaviour in a cat or dog.
